(19) Europäisches Patentamt
European Patent Office
Office européen des brevets

(11) Veröffentlichungsnummer: **0 036 134**
**A2**

(12) # EUROPÄISCHE PATENTANMELDUNG

(21) Anmeldenummer: **81101570.0**

(22) Anmeldetag: **05.03.81**

(51) Int. Cl.³: **A 61 K 7/32**

(30) Priorität: **13.03.80 DE 3009546**

(43) Veröffentlichungstag der Anmeldung:
**23.09.81 Patentblatt 81/38**

(84) Benannte Vertragsstaaten:
**AT BE CH DE FR GB IT LI LU NL SE**

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Möller, Heinrich, Dr.**
**Erlanger Strasse 45**
**D-4000 Düsseldorf 13(DE)**

(72) Erfinder: **Osberghaus, Rainer, Dr.**
**Südallee 47**
**D-4000 Düsseldorf 13(DE)**

(54) **Desodorierende kosmetische Zusammensetzungen.**

(57) Gegenstand der Erfindung sind desodorierende kosmetische Zusammensetzungen mit einem Gehalt an Derivaten mittelbis längerkettiger Alkansäuren der Formel

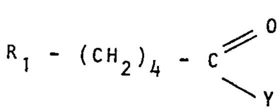

in der $R_1$ = Alkylrest mit 1 - 11 C-Atomen oder

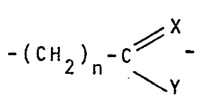 -Gruppe , wobei

n = 0-8
X = O
Y = -OH, $-OR_4$, $-NR_5R_6$, wobei
$R_4$ = Alkylrest mit 1 - 10 C-Atomen oder Aralkylrest
$R_5, R_6$ = H, Alkylrest mit 1 - 12 C-Atomen, Hydroxyalkylrest mit 2 - 4 C-Atomen, Alkoxyalkylrest, Aryl-, Aralkylrest, zusammen mit dem N ein Hetrocyclus

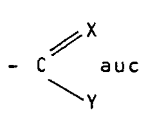 auch eine Nitrilgruppe sein kann.

Die desodorierenden Zusammensetzungen enthalten bevorzugt 0,1 - 5 Gew.% der Alkansäurederivate und 0,01 - 1 Gew.% Antioxidantien neben sonst für desodorierende Zubereitungen gebräuchlichen Komponenten.

EP 0 036 134 A2

COMPLETE DOCUMENT

Düsseldorf, den 12.3.1980

HENKEL KGaA
ZR-FE/Patente
Z-sü

# P a t e n t a n m e l d u n g

D 6097 EP

## "Desodorierende kosmetische Zusammensetzungen"

Die Erfindung betrifft desodorierende kosmetische Zusammensetzungen mit einem Gehalt an Derivaten mittel- bis längerkettiger Alkansäuren.

Es ist bekannt, daß der störende Geruch, der die Perspiration des Menschen begleitet, durch die bakterielle Zersetzung des zunächst geruchlosen Schweißes verursacht wird. Es hat daher nicht an Vorschlägen gefehlt, diesem Übelstand zu begegnen, ohne daß es bisher zu einer allseits befriedigenden Lösung gekommen wäre. Im wesentlichen handelt es sich um zwei Wege, die zur Lösung des Problems eingeschlagen wurden, einmal um den Einsatz antimikrobieller Verbindungen zur Abtötung beziehungsweise Wachstumshemmung der bakteriellen Hautflora, die die Zersetzung des Schweißes verursacht, zum anderen um den Einsatz von Verbindungen, die die Schweißabsonderung unterbinden. Daneben spielen noch rein sorptiv wirkende sowie geruchsüberdeckende Mittel eine völlig untergeordnete Rolle. Sowohl der Einsatz der die Schweißabsonderung unterbindenden Antitranspirantien als auch der antimikrobiellen Verbindungen ist umstritten, da im ersten Fall ein Eingriff in den physiologischen Vorgang der Schweißbildung erfolgt, was unerwünscht ist und im zweiten Fall neben der Wachstumshemmung der die Schweißzersetzung bewirkenden Bakterien auch ein Eingriff in die natürliche Hautflora erfolgt. Darüber hinaus kann der längere Gebrauch von Antitranspirantien zu Hautreizungen und zu Hautveränderungen führen. Bei den kosmetischen Mitteln mit desodorierender Wirkung handelt es sich durchweg um

Mittel mit einem Gehalt an antimikrobiellen Stoffen. Als solche werden zum Beispiel Phenolderivate mit und ohne Halogensubstituenten, quartäre Ammoniumverbindungen, desinfizierend wirkende Abkömmlinge von Aminosäuren vorgeschlagen. Wenn bei dem Einsatz der Deodorantien die Gefahr von Hautreizungen nicht in so hohem Maße wie bei der Verwendung von Antitranspirantien heraufbeschworen wird, so treten auch bei der laufenden Benutzung von Antimikrobika enthaltenden Deodorantien neben der Schädigung der hauteigenen Flora gelegentliche Unverträglichkeiten, Lichtsensibilisierungen und toxische Nebenwirkungen unterschiedlicher Stärke auf. Darüber hinaus ist die Mehrzahl dieser Produkte nicht geruchlos, manche besitzen einen leicht phenolischen Geruch, der bei vielen Benutzern auf Ablehnung stößt. Man ist daher weiterhin bestrebt, sehr gut desodorierende, von Nebenwirkungen weitgehend freie kosmetische Mittel zur Unterdrückung von Körpergeruch herzustellen.

Es wurde nun gefunden, daß desodorierende kosmetische Zusammensetzungen mit einem Gehalt an Derivaten mittel- bis längerkettiger Alkansäuren der allgemeinen Formel

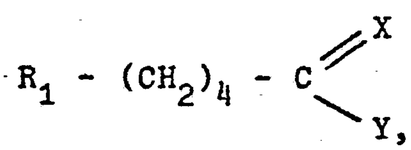

in der $R_1$ für einen Alkylrest mit 1 - 11 Kohlenstoffatomen oder eine

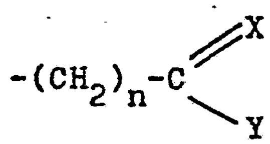

-gruppe, wobei n eine ganze Zahl von 0 bis 8 bedeutet, X für Sauerstoff, Y für -OH, $-OR_4$, $-NR_5R_6$, wobei $R_4$ einen Alkylrest mit 2 - 10 Kohlenstoffatomen oder einen Aralkylrest und $R_5$ und $R_6$ unabhängig voneinander Wasserstoff, einen Alkylrest mit 1 - 12 Kohlenstoffatomen, einen Hydroxyalkylrest mit 2 - 4 Kohlenstoffatomen, einen Alkoxyalkylrest, einen Aryl- oder Aralkylrest bedeuten oder zusammen mit dem Stickstoffatom einen heterocyclischen Ring bilden, beziehungsweise $-C\begin{smallmatrix}{=}X\\ {-}Y\end{smallmatrix}$ auch für eine Nitrilgruppe stehen, die gestellten Anforderungen weitgehend erfüllen.

Die Herstellung der erfindungsgemäß einzusetzenden Derivate mittel- bis längerkettiger Alkansäuren erfolgt nach allgemein bekannten Verfahren der organischen Synthese, z. B. der Ester durch Reaktion der Alkansäuren mit den entsprechenden Alkoholen in Gegenwart von Schwefelsäure, der Amide vorteilhaft über die entsprechenden Säurechloride und der Nitrile durch Dehydratisierung der primären Amide.

Als den erfindungsgemäß einzusetzenden Derivaten mittel- bis längerkettiger Alkansäuren zugrunde liegende Carbonsäuren sind. z.B. Capronsäure. Önanthsäure, Caprylsäure, Pelargonsäure, Caprinsäure, Laurinsäure, Myristinsäure, Palmitinsäure, Kokosfettsäure, Adipinsäure, Pimelinsäure, Korksäure, Azelainsäure, Sebacinsäure, Dodecan-1,12-disäure anzuführen.

Als Alkylreste der alkoholischen Komponente der erfindungsgemäß einzusetzenden Esterderivate mittel- bis längkettiger Alkansäuren sind zum Beispiel der Methyl-, Ethyl-, Propyl-, Isopropyl-, n-Butyl-, iso-Butyl-, sek. Butyl-, tert. Butyl-, Pentyl-, Hexyl-, 2-Ethylhexyl-, Octyl-, Nonyl-, Isononyl-, Decylrest zu nennen.

Als Amidkomponente kommen neben primären Amiden zum Beispiel Methyl-, Dimethyl-, Ethyl-, Diethyl-, Propyl-, Dipropyl-, Methylpropyl-, 2-Propyl-, Di-2-propyl-, Butyl-, Dibutyl-, sek. Butyl-, tert. Butyl-, Hexyl-, Dihexyl-, 2-Ethylhexyl-, Octyl-, Ethyloctyl-, Decyl-, Dodecyl-, Ethanol-, Diisopropanol-, 3-Methoxypropyl-, 3-(2-Ethylhexoxy)-propyl-, Benzyl-amid, Anilid, N-Methylanilid, Piperidid, 2-Methylpiperidid, 3-Methylpiperidid, 4-Methylpiperidid, 2,6-Dimethylpiperidid, 3,5-Dimethylpiperidid, Morpholid, 2,6-Dimethylmorpholid und Amid-ethylenoxidadditionsprodukte in Frage.

Als erfindungsgemäß einzusetzende Derivate mittel- bis langkettiger Alkansäuren sind zum Beispiel Capronsäureethylester, Önanthsäurebutylester, Myristinsäureoctylester, Palmitinsäurepropylester, Adipinsäuredihexylester, Dodecan-1,12-disäureisononylester, Caprylsäuredipropylamid, Caprylsäuredibutylamid, Caprinsäurediethylamid, Caprinsäuremorpholid, Caprinsäuredibutylamid, Laurinsäurediethylamid, Laurinsäuredipropylamid, Laurinsäuredibutylamid, Kokosfettsäurediethanolamid, Kokosfettsäureethanolamid + 4 Ethylenoxid zu nennen.

Zur Herstellung der erfindungsgemäßen desodorierenden kosmetischen Zusammensetzungen können die Derivate mittel- bis langkettiger Alkansäuren in alle üblicherweise für Deodorantien gebräuchlichen Zubereitungen eingearbeitet werden wie Puder, Stifte, Roll-on und Sprays, wobei der Deo-Spray das bevorzugte Einsatzgebiet ist. Die Einarbeitung erfolgt in bekannter Weise durch einfaches Vermischen oder Lösen in den anderen Komponenten der Zubereitung wie Lösungsmitteln, Wachsen, Fettsubstanzen, Polyglykolen, Pudergrundstoffen. Die erfindungsgemäßen desodorierenden kosmetischen Zusammensetzungen enthalten dabei die Derivate mittel- bis langkettiger Alkansäuren in Mengen von 0,1 bis 5 Gewichtsprozent, vorzugsweise 0,5 bis 2 Gewichtsprozent, bezogen auf die gesamte Zusammensetzung.

Die erfindungsgemäßen desodorierenden kosmetischen Zusammensetzungen werden die Derivate mittel- bis langkettiger Alkansäuren bevorzugt als alleinige Deo-Wirkstoffe enthalten, jedoch ist auch eine Kombination mit anderen desodorierenden Wirkstoffen möglich.

Die desodorierende Wirkung der erfindungsgemäßen kosmetischen Zusammensetzungen läßt sich steigern, wenn diese neben den Derivaten mittel- bis langkettiger Alkansäuren Antioxidantien in Mengen von 0,01 bis 1 Gewichtsprozent, vorzugsweise 0,05 bis 0,5 Gewichtsprozent, bezogen auf die gesamte Zusammensetzung enthalten.

Als in den erfindungsgemäßen Zusammensetzungen einzusetzende Antioxidantien sind alle auf dem pharmazeutischen, kosmetischen und Nahrungsmittel-Sektor gebräuchlichen Antioxidantien geeignet und es sind folgende Produkte zu nennen:

Butylhydroxytoluol, Butylhydroxyanisol, Guajakharz, Lecithin, Nordihydroguajaretsäure, Propylgallat, Octylgallat, Dodecylgallat, Tocopherole, Trihydroxybutyrophenon, Ascorbinsäure, Ascorbylpalimitat, Dilaurylthiodipropionat, Distearylthiodipropionat, Monoisopropylcitrat, Thiodipropionsäure und Citraconsäure.

Die nachfolgenden Beispiele sollen den Gegenstand der Erfindung näher erläutern, ohne ihn jedoch hierauf zu beschränken.

/7

## Beispiele

Zunächst werden einige Beispiele für in den erfindungsgemäßen desodorierenden kosmetischen Zusammensetzungen einzusetzende Derivate mittel- bis langkettiger Alkansäuren aufgeführt.

Produkt A: Caprylsäure-dibutylamid.

Zu einer Lösung von 90,5 g (0,7 Mol) Dibutylamin in 200 ml Ether wurden unter Rühren und Kühlen 48,8 g (0,3 Mol) Cyprylsäurechlorid getropft. Danach wurde 2 Stunden bei Raumtemperatur und 1 Stunde bei Siedetemperatur gerührt, der Niederschlag abgesaugt, mit Ether nachgewaschen, die Lösung mit verdünnter Salzsäure, verdünnter Sodalösung und Wasser gewaschen und der Eindampfrückstand unter vermindertem Druck destilliert. Es wurden 47,5 g Caprylsäure-dibutylamid vom Siedepunkt 96-97° C/0,07 mbar und einem Brechungsindex $n_D^{20}$: 1,4568 erhalten.

In analoger Weise wurden die nachstehenden Amide hergestellt.

Produkt B: Caprylsäure-dipropylamid, Sdp. 104° C/0,17 mbar, $n_D^{20}$: 1,4510.

Produkt C: Caprinsäure-diethylamid, Sdp. 93° C/0,13 mbar, $n_D^{20}$: 1,4522.

Produkt D: Caprinsäure-dipropylamid, Sdp. 113° C/0,13 mbar $n_D^{20}$: 1,4533.

Produkt E: Caprinsäure-morpholid; Sdp. 123-124° C/0,04 mbar, $n_D^{20}$: 1,4734.

**Produkt F:** Caprinsäure-dibutylamid: Sdp. 147° C/ 0,13 mbar, $n_D^{20}$: 1,4545.

**Produkt G:** Laurinsäure-diethylamid: Sdp. 118° C/ 0,13 mbar, $n_D^{20}$: 1,4539.

**Produkt H:** Laurinsäure-dipropylamid: Sdp. 132° C/ 0,13 mbar $n_D^{20}$: 1,4553.

**Produkt J:** Laurinsäure-dibutylamid: Sdp. 145° C/ 0,13 mbar, $n_D^{20}$: 1,4516.

Weiterhin wurden folgende Handelsprodukte in den Beispielen eingesetzt:

**Produkt K:** Kokosfettsäure-diethanolamid, Comperlan KD(R) (Dehydag).

**Produkt L:** Kokosfettsäure-ethanolamid + 4 EO, Eumulgin C 4(R) (Dehydag).

**Produkt M:** Dodecandisäure-diisononylester, Drivolan D9(R) (Chemische Werke Hüls).

**Produkt N:** Sebacinsäure-dibutylester, Sdp. 344-345° C/ $n_D^{20}$: 1,443.

**Produkt O:** Sebacinsäure-bis-2-ethylhexylester, Sdp. 256° C/ 7 mbar, $n_D^{20}$: 1,451.

/9

Nachstehend werden einige Beispiele für erfindungsgemäße desodorierende kosmetische Zusammensetzungen angegeben.

Desodorierender Stift

| | |
|---|---|
| 2-Octyldodecanol | 27,0 Gewichtsteile |
| Cetylstearylalkohol | 4,0 " |
| Natriumstearat | 9,0 " |
| Kokosfettsäuremonoethanolamid | 3,0 " |
| Paraffinöl | 4,0 " |
| Propylenglykol | 2,0 " |
| Ethanol | 49,0 " |
| Produkt A | 2,0 " |

Desodorierender Puder

| | |
|---|---|
| Reisstärke | 27,0 Gewichtsteile |
| Magnesiumcarbonat | 3,0 " |
| Zinkoxid | 2,0 " |
| Talkum extra fein | 81,0 " |
| Produkt C | 2,0 " |

Desodorierender Spray

| | |
|---|---|
| Produkt E | 1,5 Gewichtsteile |
| Ethanol | 11,0 " |
| Isopropanol | 20,5 " |
| Isopropylmyristat | 2,0 " |
| Treibgas Frigen 12/114 60:40 | 65,0 " |

Desodorierender Spray

| | |
|---|---|
| Produkt G | 1,3 Gewichtsteile |
| Butylhydroxytoluol | 0,2 " |
| Propylenglykol | 2,5 " |
| Isopropylmyristat | 2,0 " |
| Ethanol | 14,0 " |
| Treibgas Frigen 11/12 50:50 | 80,0 " |

Deodorant-Creme

| | | |
|---|---|---|
| Gemisch von Mono- und Diglyceriden der Palmitin- und Stearinsäure, Cutina MD(R) Dehydag | 20,5 | Gewichtsteile |
| Cetyl/Stearylalkohol + ca. 12 Mol Ethylenoxid Eumulgin B1(R) Dehydag | 2,0 | " |
| Produkt L | 1,3 | " |
| Butylhydroxytoluol | 0,2 | " |
| Wasser | 61,9 | " |
| p-Hydroxybenzoesäuremethylester | 0,1 | " |
| Parfümöl | 1,0 | " |

Deodorans für Pumpzerstäuber

| | | |
|---|---|---|
| Ethanol | 85,0 | Gewichtsteile |
| Isopropanol | 7,2 | " |
| Produkt M | 1,5 | " |
| Butylhydroxytoluol | 0,3 | " |
| Parfüm | 1,0 | " |
| Wasser | 5,0 | " |

An die Stelle der genannten Produkte können mit gleichem Erfolg die vorstehend aufgeführten Verbindungen treten.

Für eine vergleichende Wirksamkeitsprüfung wurde der folgende desodorierende Spray hergestellt.

Desodorierender Spray A

| | | |
|---|---|---|
| Produkt A | 1,5 | Gewichtsteile |
| Isopropanol | 5,5 | " |
| Ethanol | 33,0 | " |
| Treibgas Frigen 12/114 60:40 | 60,0 | " |

Vergleichsspray B

| | | |
|---|---|---|
| Cypryl-/Caprinsäuretriglycerid | 1,5 | Gewichtsteile |
| Isopropanol | 5,5 | " |
| Ethanol | 33,0 | " |
| Treibgas Frigen 12/114 60:40 | 60,0 | " |

Eine Testgruppe, bestehend aus 15 weiblichen und 15 männlichen Teilnehmern, benutzte zunächst 5 Tage lang eine von antimikrobiellen Mitteln freie Seife F und keine Deodorantien oder Antiperspirantien. Nach dieser Zeit erhielt jeder Teilnehmer ein T-Shirt und die Instruktion, am 6. Tage morgens nach der Wäsche mit der Seife F eine Achsel mit dem Deo-Spray A zu behandeln und die andere Achsel zum Vergleich nicht zu behandeln, wobei die eine Hälfte der Gruppe die linke Achsel, die andere Hälfte die rechte Achsel behandelte. Die Geruchsentwicklung wurde von den Versuchspersonen selbst sowie von zwei erfahrenen Kosmetikern durch Abriechen der T-Shirts nach 8 Stunden und 24 Stunden beurteilt. Hiernach wurde von den Versuchspersonen eine Woche lang lediglich die Seife F benutzt.Anschließend wurde der Test wiederholt, wobei die zuvor unbehandelte Achsel mit dem Deo-Spray A behandelt wurde und die andere Achsel zum Vergleich diente.

In beiden Tests wurde von allen am Versuch beteiligten Personen eine sehr gute Geruchsunterbindung durch den desodorierenden Spray A festgestellt.

Mit der gleichen Testgruppe wurde der Test in völlig analoger Form wiederholt, nur daß an Stelle des Deo-Sprays A jeweils der Vergleichsspray B eingesetzt wurde.

Bei diesem Versuch konnte von keiner der am Versuch beteiligten Personen eine signifikante Geruchsminderung festgestellt werden.

/12

Patentansprüche:

1. Desodorierende kosmetische Zusammensetzungen, gekennzeichnet durch einen Gehalt an Derivaten mittel- bis längerkettiger Alkansäuren der allgemeinen Formel

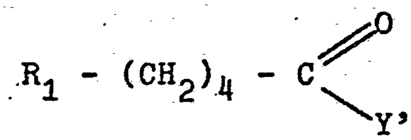

in der $R_1$ für einen Alkylrest mit 1 - 11 Kohlenstoffatomen oder eine 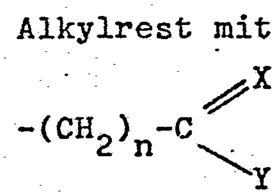 -gruppe, wobei n eine ganze Zahl von 0 bis 8 bedeutet, X für Sauerstoff, Y für -OH, $-OR_4$, $-NR_5R_6$, wobei $R_4$ einen Alkylrest mit 2 - 10 Kohlenstoffatomen oder einen Aralkylrest und $R_5$ und $R_6$ unabhängig voneinander Wasserstoff, einen Alkylrest mit 1 - 12 Kohlenstoffatomen, einen Hydroxyalkylrest mit 2 - 4 Kohlenstoffatomen, einen Alkoxyalkylrest, einen Aryl- oder Aralkylrest bedeuten oder zusammen mit dem Stickstoffatom einen heterocyclischen Ring bilden, beziehungsweise

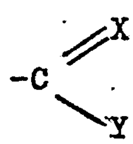 auch für eine Nitrilgruppe stehen.

2. Desodorierende kosmetische Zusammensetzungen nach Anspruch 1, dadurch gekennzeichnet, daß sie die Derivate mittel- bis langkettiger Alkansäuren in einer Menge von 0,1 bis 5 Gewichtsprozent, vorzugsweise 0,5 bis 2 Gewichtsprozent, bezogen auf die gesamte Zusammensetzung, enthalten.

3. Desodorierende kosmetische Zusammensetzungen nach Anspruch 1 und 2, dadurch gekennzeichnet, daß sie neben den Derivaten mittel- bis langkettiger Alkansäuren Antioxidantien in einer Menge von 0,01 bis 1 Gewichtsprozent, vorzugsweise 0,05 bis 0,5 Gewichtsprozent, bezogen auf die gesamte Zusammensetzung enthalten.